(19) Europäisches Patentamt
European Patent Office
Office européen des brevets

(11) Veröffentlichungsnummer : **0 148 442**
**B1**

(12) # EUROPÄISCHE PATENTSCHRIFT

(45) Veröffentlichungstag der Patentschrift : **21.01.87**

(51) Int. Cl.[4] : **C 07 C149/237**, C 07 C149/20, C 07 C149/23, A 01 N 37/36

(21) Anmeldenummer : **84115220.0**

(22) Anmeldetag : **12.12.84**

(54) **Halogenierte Sulfide, Verfahren zu ihrer Herstellung und ihre Verwendung in mikrobiziden Mitteln.**

(30) Priorität : **24.12.83 DE 3346947**

(43) Veröffentlichungstag der Anmeldung : **17.07.85 Patentblatt 85/29**

(45) Bekanntmachung des Hinweises auf die Patenterteilung : **21.01.87 Patentblatt 87/04**

(84) Benannte Vertragsstaaten : **AT BE CH DE FR GB IT LI NL SE**

(56) Entgegenhaltungen :
**EP-A- 0 001 312**
**US-A- 2 762 836**
**US-A- 2 993 037**

(73) Patentinhaber : **BAYER AG**
**Konzernverwaltung RP Patentabteilung**
**D-5090 Leverkusen 1 Bayerwerk (DE)**

(72) Erfinder : **Oeckl, Siegfried, Dr.**
**Auf der Höhe 74**
**D-5060 Bergisch-Gladbach 1 (DE)**
Erfinder : **Schade, Gerold, Dr.**
**Hahnenweg 8**
**D-5000 Köln 80 (DE)**
Erfinder : **Schmitt, Hans-Georg, Dr.**
**Gustav-Freytag-Strasse 2**
**D-5090 Leverkusen 1 (DE)**
Erfinder : **Paulus, Wilfried, Dr.**
**Deswatinesstrasse 90**
**D-4150 Krefeld 1 (DE)**
Erfinder : **Genth, Hermann, Dr.**
**Am Heckerhof 60**
**D-4150 Krefeld 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 148 442 B1**

Jouve, 18, rue St-Denis, 75001 Paris, France

**Beschreibung**

Die Erfindung betrifft neue halogenierte Sulfide, Verfahren zu ihrer Herstellung und ihre Verwendung in mikrobiziden Mitteln.

Es wurden neue halogenierte Sulfide der Formel

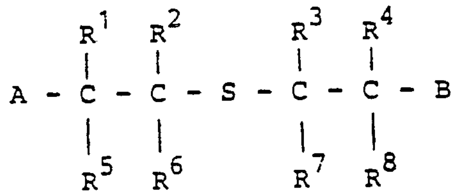

(I)

in der

$R^1$ bis $R^8$ gleich oder verschieden sind und Wasserstoff oder Halogen bedeuten, wobei mindestens einer der Reste $R^1$ bis $R^8$ für Halogen steht, und wobei gegebenenfalls $R^1$ mit $R^2$ und/oder $R^3$ mit $R^4$ eine olefinische Bindung bilden können, und

A und B gleich oder verschieden sind und Nitril, Carboxyl, Carboxylat oder Carboxamid bedeuten, gefunden.

Soweit möglich können die neuen Verbindungen in Form ihrer verschiedenen Stereoisomeren vorliegen.

Halogen bedeutet erfindungsgemäß Chlor, Brom oder Jod, bevorzugt Chlor und Brom ; insbesondere bevorzugt wird Chlor.

Unter Carboxyl im Rahmen der vorliegenden Erfindung wird die Gruppe —COOH verstanden.

Unter Carboxylat im Rahmen der vorliegenden Erfindung wird die Gruppe —COOM verstanden, wobei M im allgemeinen Alkali (z. B Natrium, Kalium), Erdalkali (z. B. Magnesium, Calcium) oder Ammonium, bedeuten kann.

Unter Carboxamid im Rahmen der vorliegenden Erfindung wird die Gruppe $—CONH_2$ verstanden.

Bevorzugte halogenierte Sulfide im Rahmen der Formel I sind erfindungsgemäß Verbindungen der Formel

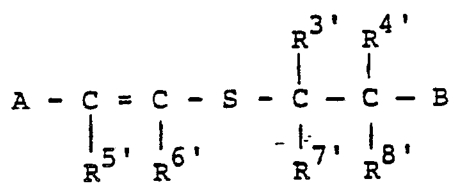

(II)

in der

$R^{3'}$ bis $R^{8'}$ gleich oder verschieden sind und Wasserstoff, Chlor oder Brom bedeuten, wobei mindestens einer der Reste $R^{3'}$ bis $R^{8'}$ für Chlor oder Brom steht und wobei $R^{3'}$ mit $R^{4'}$ gegebenenfalls eine olefinische Bindung bilden kann, und

A und B gleich oder verschieden sind und Nitril, Carboxyl, Carboxylat oder Carboxamid bedeuten.

Insbesondere werden erfindungsgemäß halogenierte Sulfide der Formel

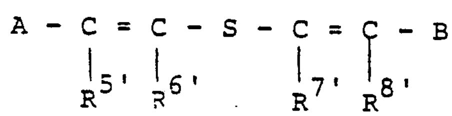

(III)

in der $R^{5'}$ bis $R^{8'}$, A und B die oben genannte Bedeutung haben, bevorzugt.

Insbesondere werden bevorzugt halogenierte Sulfide der Formel (III), in denen A und B für Nitrilgruppen stehen und in denen Halogen Chlor bedeutet.

Beispielsweise seien die folgenden halogenierten Sulfide genannt :

(1-Chlor-2-cyano-vinyl)-(2-cyano-ethyl)-sulfid,
(1,2-Dichlor-2-cyano-vinyl)-(2-cyano-ethyl)-sulfid,
(1-Chlor-2-cyano-vinyl)-(2,2-dichlor-2-cyano-ethyl)-sulfid,
(1,2-Dichlor-2-cyano-vinyl)-2,2-dichlor-2-cyano-ethyl)-sulfid,
(1,2-Dichlor-2-cyano-vinyl)-(2-chlor-2-cyano-vinyl)-sulfid
Bis-(2-chlor-2-cyano-vinyl)-sulfid,
Bis-(1,2,2-trichlor-2-cyano-ethyl)-sulfid,

Bis-(1,2-dichlor-2-cyano-vinyl)-sulfid,
(2-Brom-2-cyano-vinyl)-(2-cyano-ethyl)-sulfid,
(2-Brom-1-chlor-2-cyano-vinyl)-(2-cyano-ethyl)-sulfid, 2,2-Dichlor-3-(1,2-dichlor-2-cyano-vinyl)-thiopropionsäureamid,
2,2-Dichlor-3-(1,2-dichlor-2-cyano-vinyl)-thio-propionsäure und deren Natriumsalze.

Es wurden mehrere Verfahren zur Herstellung der neuen halogenierten Sulfide gefunden. So wurde ein Verfahren zur Herstellung von halogenierten Sulfiden der Formel

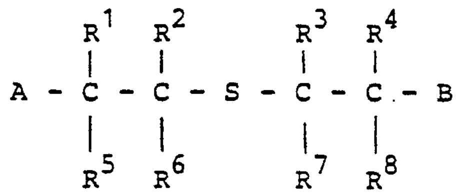

(I)

in der

$R^1$ bis $R^8$ gleich oder verschieden sind und Wasserstoff oder Halogen bedeuten, wobei mindestens einer der Reste $R^1$ bis $R^8$ für Halogen steht, und wobei gegebenenfalls $R^1$ mit $R^2$ und/oder $R^3$ mit $R^4$ eine olefinische Bindung bildet, und

A und B gleich oder verschieden sind und Nitril, Carboxyl, Carboxylat oder Carboxamid bedeuten,

gefunden, das dadurch gekennzeichnet ist, daß man Bis-(cyanethyl)-sulfid mit einem Halogenierungsmittel, gegebenenfalls in Gegenwart eines säurebindenden Mittels, umsetzt und dann in an sich bekannter Weise aufarbeitet.

Das erfindungsgemäße Verfahren kann durch die folgende Reaktionsgleichung erläutert werden :

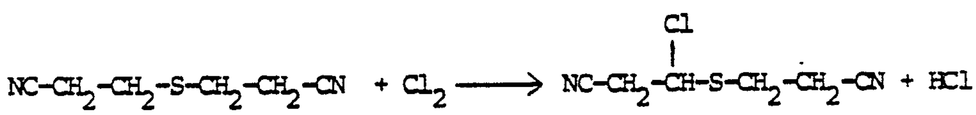

Nach dem erfindungsgemäßen Verfahren erhält man sowohl gesättigte als auch ungesättigte erfindungsgemäße halogenierte Sulfide. In den meisten Fällen erhält man ein Gemisch aus verschiedenen Verbindungen. Die reinen Verbindungen erhält man leicht aus dem Gemisch durch an sich bekannte Trennmethoden wie Kristallisation, Gaschromatographie, Flüssigkeitschromatographie, Dünnschichtchromatographie oder Destillation.

Als Halogenierungsmittel finden die freien Halogene wie Chlor, Brom und Jod, vorzugsweise Chlor oder Brom, sowie Sulfurylhalogenide wie Sulfurylchlorid und Phosphorhalogenide wie Phosphorpentachlorid Verwendung.

Das erfindungsgemäße Verfahren kann ohne Lösungsmittel durchgeführt werden oder in Gegenwart solcher Lösungsmittel, die gegen das verwendete Halogenierungsmittel inert sind. Als solche kommen beispielsweise niedere aliphatische Carbonsäuren wie Essigsäure oder Chlorkohlenwasserstoffe wie Dichlormethan, Trichlormethan, Dichlorethan oder Chlorbenzol in Frage. Bevorzugte Lösungsmittel sind Dichlorkohlenwasserstoffe wie Dichlormethan, 1,2-Dichlorethan und Dichlorbenzol.

Die Reaktionstemperatur des erfindungsgemäßen Verfahrens richtet sich nach dem verwendeten Halogenierungsmittel und liegt im allgemeinen im Bereich von —40 bis + 120 °C, bevorzugt im Bereich von —20 °C und + 80 °C. Es kann zweckmäßig sein, das erfindungsgemäße Verfahren bei niedrigen Temperaturen zu beginnen und die Temperatur im Verlaufe der Halogenierung zu erhöhen.

Das erfindungsgemäße Verfahren wird im allgemeinen bei Normaldruck durchgeführt. Es ist jedoch auch möglich das Verfahren bei einem Unter- oder Überdruck (beispielsweise im Druckbereich von 0,1 bis 100 bar) durchzuführen.

Das erfindungsgemäße Verfahren kann gegebenenfalls in Gegenwart eines säurebindenden Mittels durchgeführt werden. Im allgemeinen ist es zweckmäßig säurebindende Mittel zuzusetzen, um im wesentlichen erfindungsgemäße ungesättigte halogenierte Sulfide zu erhalten. Säurebindende Mittel für das erfindungsgemäße Verfahren sind beispielsweise Hydroxide, Carbonate, Hydrogencarbonate und Acetate von Alkali-(beispielsweise Natrium und Kalium) und Erdalkalimetallen (beispielsweise Magnesium und Calcium).

Säurebindende Mittel für das erfindungsgemäße Verfahren können auch aliphatische oder aromatische tertiäre Amine wie Triethylamin, Dimethylanilin, Ethyldicyclohexylamin oder Heterocyclen wie Pyridin oder Chinolin sein.

Selbstverständlich ist es auch möglich Gemische der säurebindenden Mittel einzusetzen. Im allgemeinen setzt man sie erfindungsgemäß entsprechend der abzuspaltenden Menge Halogenwasserstoff in stöchiometrischer Menge ein. Es ist jedoch auch möglich sie im Überschuß oder Unterschuß

(beispielsweise im Verhältnis 0,01 bis 2, bevorzugt 0,01 bis 0,5 bezogen auf das Bis(cyanethyl) sulfid, einzusetzen.

Es kann vorteilhaft sein das erfindungsgemäße Verfahren in Gegenwart eines Phasentransferkatalysators durchzuführen. Phasentransferkatalysatoren sind hierbei beispielsweise Tetraalkylammoniumsalze und Tetraalkylphosphoniumsalze (wobei Alkyl für einen Alkylrest mit 1 bis etwa 16 Kohlenstoffatomen steht) oder Polyether (wobei sowohl ringförmige als auch lineare langkettige Polyether mit der wiederkehrenden Einheit [$-CH_2-CH_2-O$] in Betracht kommen).

In einer besonderen Ausführungsform des erfindungsgemäßen Verfahrens setzt man in einer ersten Stufe das Bis-(cyanethyl)-sulfid mit dem Halogenierungsmittel um und fügt dann in einer zweiten Stufe das säurebindende Mittel zu.

Im allgemeinen steuert man den Halogenierungsgrad der erfindungsgemäßen neuen halogenierten Sulfide durch die Menge des zugegebenen Halogenierungsmittels. Man führt daher erfindungsgemäß je nach Anzahl der einzuführenden Halogene äquivalente Mengen des Halogenierungsmittels zu.

Es ist aber auch möglich, den Reaktionsfortgang mit analytischen Mitteln zu verfolgen ; als solche kommen Gaschromatographie, Flüssigchromatographie und Dünnschichtchromatographie in Betracht. Dadurch ist es möglich, die Reaktion nach Zugabe bestimmter Mengenhalogenierungsmittel abzubrechen, wodurch der Halogengehalt der Produkte gesteuert werden kann.

In einer anderen Ausführungsform des erfindungsgemäßen Verfahrens erhält man besonders reine höherhalogenierte Sulfide, wenn man in einem ersten Schritt erfindungsgemäß ungesättigte halogenierte Sulfide herstellt und dann in einem zweiten Schritt gegebenenfalls auch ein anderes Halogen anlagert.

Das erfindungsgemäße Verfahren kann diskontinuierlich aber auch kontinuierlich durchgeführt werden.

Im allgemeinen wird das erfindungsgemäße Verfahren so durchgeführt, daß man die Ausgangsverbindungen in dem gegebenenfalls verwendeten Lösungsmittel löst und das entsprechende Halogenierungsmittel bei der erfindungsgemäßen Reaktionstemperatur zugibt. Die Zugabe kann auf einmal, portionsweise oder kontinuierlich erfolgen ; so werden gasförmige und flüssige Halogenierungsmittel oft vorteilhaft in kontinuierlicher Form zugesetzt.

Nach Beendigung der Umsetzung werden gegebenenfalls Lösungsmittel, überschüssiges Halogenierungsmittel und säurebindende Mittel abgetrennt und das Produkt durch übliche Verfahren wie Destillation und Kristallisation gereinigt. Es kann auch zweckmäßig sein, das erfindungsgemäß halogenierte Sulfid durch Chromatographie von Verunreinigungen zu befreien.

Es ist überraschend, daß das erfindungsgemäße Verfahren zur Herstellung der erfindungsgemäßen halogenierten Sulfide benutzt werden kann. Denn bekanntlich werden Kohlenstoff Schwefel-Bindungen durch Halogenierungsmittel sehr leicht gespalten. (Tetrahedron *38*, 2612 ff (1982)).

Erfindungsgemäße neue halogenierte Sulfide der Formel

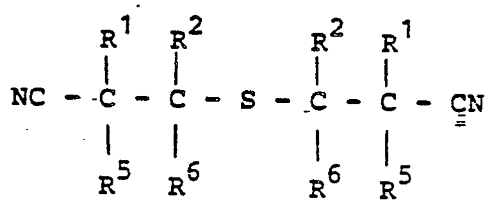

(Ia)

in der $R^1$, $R^2$, $R^5$ und $R^6$ gleich oder verschieden sind und Wasserstoff oder Halogen bedeuten, wobei mindestens einer der Reste $R^1$, $R^2$, $R^5$ oder $R^6$ für Halogen steht, und wobei gegebenenfalls $R^1$ mit $R^2$ eine olefinische Bindung bilden kann, können auch hergestellt werden, indem man in einem ersten Reaktionsschritt ein Sulfid der Formel

$$Me_{2/n}S \quad (IV),$$

in der

Me Wasserstoff, Alkali, Erdalkali oder Ammonium bedeutet und

n für die Wertigkeit des Restes Me steht,

mit 3-Halogen-acrylnitrilen der Formel

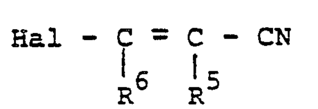

(V)

in der

Hal für Chlor oder Brom steht und

$R^5$ und $R^6$ die obengenannte Bedeutung haben,

gegebenenfalls in Gegenwart einer Base umsetzt, dann gegebenenfalls in einem zweiten Reaktionsschritt das Reaktionsprodukt halogeniert, dann gegebenenfalls in einem dritten Reaktionsschritt mit einem

säurebindenden Mittel umsetzt und dann gegebenenfalls in einem vierten Reaktionsschritt wieder halogeniert und die einzelnen Reaktionsschritte gegebenenfalls in an sich bekannter Weise aufarbeitet.

In der ersten Stufe des erfindungsgemäßen Verfahrens erhält man erfindungsgemäße Verbindungen der Formel

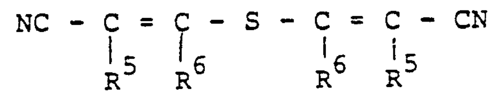

(VI)

in der $R^5$ und $R^6$ die obengenannte Bedeutung haben.

An diese Verbindungen der Formel (VI) kann gegebenenfalls in der zweiten Reaktionsstufe Halogen angelagert werden. Man erhält dann Verbindungen der Formel

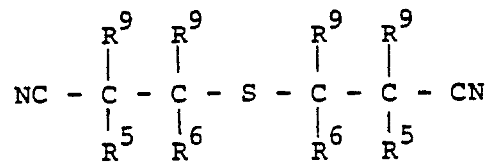

(VII)

in der

$R^5$ und $R^6$ die obengenannte Bedeutung haben und

$R^9$ für Halogen steht.

Sofern in der Formel (VII) $R^5$ oder $R^6$ für Wasserstoff steht, ist es möglich in dem dritten Reaktionsschritt in Gegenwart eines säurebindenden Mittels Halogenwasserstoff abzuspalten. Man erhält dann wieder erfindungsgemäße halogenierte Sulfide mit olefinischer Bindung.

Durch gegebenenfalls nochmalige Anlagerung von Halogen an die so entstandene olefinische Bindung erhält man erfindungsgemäße perhalogenierte Verbindungen.

Durch diese erfindungsgemäße Verfahrensweise ist es möglich, durch Verwendung von verschiedenen Halogenen gemischt halogenierte Verbindungen zu erhalten.

Das erfindungsgemäße Verfahren kann durch die folgenden Reaktionsgleichungen erläutert werden :

$$2\ CN{-}CCl{=}CHCl + Na_2S \xrightarrow{-2NaCl} CN{-}CCl{=}CH{-}S{-}CH{=}CCl{-}CN$$

$$CN{-}CCl{=}CH{-}S{-}CH{=}CCl{-}CN + Cl_2 \longrightarrow CN{-}CCl_2{-}CHCl{-}S{-}CHCl{-}CCl_2{-}CN$$

$$CN{-}CCl_2{-}CHCl{-}S{-}CHCl{-}CCl_2{-}CN \xrightarrow{-2HCl} CN{-}CCl{=}CCl{-}S{-}CCl{=}CCl{-}CN$$

$$CN{-}CCl{=}CCl{-}S{-}CCl{=}CCl{-}CN + Br_2 \longrightarrow CN{-}CClBr{-}CClBr{-}S{-}CClBr{-}CClBr{-}CN$$

Überraschenderweise ist es auch möglich, im Rahmen des erfindungsgemäßen Verfahrens zwei oder mehrere der Reaktionsschritte zusammenzufassen und in Form einer Eintopfreaktion durchzuführen.

Als Sulfide seien Sulfide und Hydrogensulfide von Alkali- (z. B. Natrium und Kalium) und Erdalkalimetallen (z. B. Magnesium und Calcium) und Ammonium, sowie Schwefelwasserstoff genannt.

3-Halogenacrylnitrile für das erfindungsgemäße Verfahren sind an sich bekannt (Angew. Chemie. *60A*. 311 (1948)). Sie können beispielsweise hergestellt werden, indem man Acrylnitril mit Halogenierungsmitteln wie Chlor gegebenenfalls in Gegenwart halogenwasserstoffabspaltender Mittel umsetzt.

Beispielsweise seien die folgenden 3-Halogenacrylnitrile genannt :

2,3-Dichloracrylnitril
2,3,3-Trichloracrylnitril
2-Chlor-3-brom-acrylnitril
2,3-Dichlor-3-brom-acylnitril.

Im allgemeinen setzt man das Sulfid und die 3-Halogenacrylnitrile in etwa stöchiometrischen Mengen ein.

Die Umsetzung wird bevorzugt in einem Lösungsmittel durchgeführt, das sich unter den Reaktionsbedingungen nicht verändert. Als solche kommen Wasser aliphatische Alkohole wie Methanol und Ethanol, Ether wie Diethylether und Tetrahydrofuran, Chlorkohlenwasserstoffe wie Dichlormethan und Tetrachlorkohlenstoff, Ketone wie Aceton, Ester wie Essigsäureethylester und Formamide wie Dimethylformamid in Frage.

Das erfindungsgemäße Verfahren wird im allgemeinen im Temperaturbereich von — 20 bis + 60 °C,

bevorzugt von 0 °C bis 40 °C durchgeführt.

Das erfindungsgemäße Verfahren wird im allgemeinen bei Normaldruck durchgeführt. Es ist jedoch auch möglich das Verfahren bei einem Überdruck (beispielsweise im Druckbereich von 1 bis 50 bar) durchzuführen.

Der erste Reaktionsschritt wird im allgemeinen wie folgt durchgeführt :

Zur Umsetzung werden die Reaktionskomponenten in Lösung bei der erfindungsgemäßen Reaktionstemperatur zusammengegeben. Das Ende der Umsetzung kann auf chromatographischem Wege festgestellt werden. Das Reaktionsprodukt wird nach allgemein üblichen Reaktionsverfahren, z. B. durch Destillation, Kristallisation oder Säulenchromatographie, aufgearbeitet.

Es ist überraschend, daß der erste Schritt des erfindungsgemäßen Verfahrens mit hohen Ausbeuten abläuft. Es ist nämlich bekannt, daß Acrylnitrile, die in 2-Position ein Halogen tragen, mit Sulfiden Thiirane bilden, die in sehr unübersichtlicher Weise reagieren (J. Org. Chem. *34,* 2955 (1969)).

Die Halogenierung nach dem zweiten und vierten Reaktionsschritt des erfindungsgemäßen Verfahrens wird im allgemeinen in Lösung durchgeführt. Zweckmäßigerweise verwendet man inerte Lösungsmittel wie z. B. $CH_2Cl_2$, $CCl_4$, $ClC_6H_5$ und Essigsäure.

Als Halogenierungsmittel finden die freien Halogene wie Chlor, Brom und Jod, vorzugsweise Chlor oder Brom, sowie Sulfurylhalogenide wie Sulfurylchlorid und Phosphorhalogenide wie Phosphorpentachlorid Verwendung.

Die Halogenierung wird im allgemeinen im Temperaturbereich von — 20 bis + 60 °C, bevorzugt von 0 bis 45 °C durchgeführt.

Im allgemeinen führt man die Halogenierung bei Normaldruck durch. Es ist aber auch möglich, die Halogenierung bei einem Überdruck (beispielsweise im Druckbereich von 1 bis 50 bar) durchzuführen.

Die Abspaltung von Halogenwasserstoff nach dem dritten Reaktionsschritt wird in Gegenwart von säurebindenden Mitteln durchgeführt.

Als säurebindende Mittel seien hier aliphatische, cycloaliphatische und aromatische tertiäre Amine genannt. Als aliphatische Gruppen seien beispielsweise geradkettige oder verzweigte Niederalkylgruppen mit 1 bis etwa 6 Kohlenstoffatomen genannt. Als cycloaliphatische Gruppen seien beispielsweise der Cyclopentyl- und der Cyclohexylrest genannt. Als aromatische Gruppen seien beispielsweise der Phenyl, Diphenyl und Naphthylrest genannt. Als tertiäre Amine seien beispielsweise Triethylamin, Dimethylanilin und Ethyldicyclohexylamin genannt. Es ist auch möglich Heterocyclen wie Pyridin und Chinolin einzusetzen. Außerdem können auch Hydroxide, Carbonate, Hydrogencarbonate und Acetate von Alkali- (z. B. Natrium und Kalium) und Erdalkalimetallen (z. B. Magnesium und Calcium) verwendet werden.

Zweckmäßigerweise werden das säurebindende Mittel und das Lösungsmittel aufeinander abgestimmt. Bei Verwendung von tertiären Aminen kommen sowohl polare als auch unpolare Lösungsmittel in Frage. Beispielsweise seien hier die folgenden Lösungsmittel genannt : Dichlormethan, Tetrachlorkohlenstoff, Toluol, Dimethylformamid, Ethanol, Aceton, Tetrahydrofuran.

Bei Verwendung von Metallsalzen werden polare Lösungsmittel bevorzugt, die 50 bis 100 Gew.-% Wasser enthalten können.

Die Temperatur des erfindungsgemäßen Reaktionsschritts liegt im allgemeinen im Bereich von — 20 °C bis 80 °C, bevorzugt im Bereich von — 10 bis + 40 °C.

Der Reaktionsschritt kann beispielsweise wie folgt durchgeführt werden :

Die Reaktionskomponenten werden in Lösung zusammengegeben und bei der erfindungsgemäßen Umsetzungstemperatur bis zum Ende der Reaktion gerührt. Das Ende der Reaktion kann durch chromatographische Verfahren festgestellt werden. Die Produkte können auf üblichem Wege, z. B. durch Destillation, Kristallisation oder Säulenchromatographie gereinigt werden.

Die nach den erfindungsgemäßen Verfahren hergestellten Nitrile können zu den entsprechenden Carboxamiden und Carbonsäuren hydrolysiert werden.

Hierzu werden sie mit einem Überschuß von konzentrierten Säuren wie Salzsäure, Schwefelsäure oder Essigsäure erhitzt.

Wird hierbei bei Temperaturen von 60 °C bis Rückflußtemperatur gearbeitet, so entstehen nach längerer Reaktionszeit (z. B. 2-10 h) die Carbonsäuren.

Unter milderen Bedingungen (Temperatur etwa 40-80 °C, Reaktionszeit etwa 0,1-2 h) entstehen die Carboxamide. Hierbei kann es zweckmäßig sein, den Reaktionsverlauf z. B. durch Dünnschichtchromatographie zu verfolgen, um die Hydrolyse nach Erreichen der Amidstufe abzubrechen.

Aus den Carbonsäuren erhält man die Metallcarboxylate durch Umsetzung mit Metallhydroxiden und -carbonaten.

Die erfindunsgemäßen halogenierten Sulfide können als Wirkstoffe zur Bekämpfung von Mikroorganismen, im besonderen in technischen Materialien, die von Mikroorganismen befallen und zersetzt werden können, verwendet werden.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vor einer mikrobiellen Veränderung oder Zerstörung geschützt werden sollen, Klebstoffe, Leime, Papier und Karton, Textilien, Leder, Holz, Anstrichmittel und Kunsstoffartikel, Kühlschmiermittel und andere Materialien sein, die von Mikroorganismen befallen und zersetzt werden können. Im Rahmen der zu schützenden Materialien seien auch Teile von Produktionsanlagen,

beispielsweise Kühlwasser- und Papiermaschinenkreisläufe genannt, die durch Mikroorganismen beeinträchtigt werden können. Im Rahmen der vorliegenden Erfindung seien als technische Materialien vorzugsweise Klebstoffe, Leime, Papier, Karton, Leder, Holz, Anstrichmittel, Kühlschmiermittel, Wasserkreisläufe, insbesondere in der Papierindustrie, genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Substanzen gegen Bakterien, Schimmelpilze, insbesondere holzverfärbende und holzzerstörende Pilze (Basidiomyceten), sowie gegen Schleimorganismen und Algen.

Es seien beispielsweise Mikroorganismen der folgenden Gattungen genannt :

Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Staphylococcus, wie Staphylococcus aureus,
Escherichia, wie Escherichia coli,
ferner Grün-, Blau-, Braun- und Kieselalgen.

Je nach ihrem Anwendungsgebiet können die erfindungsgemäßen Wirkstoffe in die üblichen Formulierungen überführt werden, wie Lösungen, Emulsionen, Suspensionen, Pulver, Pasten und Granulate.

Diese können in an sich bekannter Weise hergestellt werden, z. B. durch Vermischen der Wirkstoffe mit einem Streckmittel, das aus flüssigem Lösungsmittel und/oder Festträgerstoffen besteht, gegebenenfalls unter Verwendung von oberflächenaktiven Mitteln, wie Emulgatoren und/oder Dispergiermitteln, wobei beispielsweise im Falle der Benutzung von Wasserstreckmitteln, gegebenenfalls organische Lösungsmittel wie Alkohole, als Hilfsmittel verwendet werden können.

Organische Lösungsmittel für die Wirkstoffe können beispielsweise Alkohole, wie niedere aliphatische Alkohole, vorzugsweise Ethanol oder Isopropanol, oder Benzylalkohole, Ketone wie Aceton oder Methylethylketon, flüssige Kohlenwasserstoffe, wie Benzinfraktionen, halogenierte Kohlenwasserstoffe wie 1,2-Dichlorethan, sein.

Die Anwendungskonzentration der erfindungsgemäßen Wirkstoffe richtet sich nach der Art und dem Vorkommen der zu bekämpfenden Mikroorganismen, sowie nach der Zusammensetzung des zu schützenden Materials. Die optimale Einsatzmenge kann durch Testreihen ermittelt werden.

Im allgemeinen liegen die Anwendungskonzentrationen im Bereich von 0,001 bis 5 Gew.-%, vorzugsweise von 0,005 bis 0,5 Gew.-%, bezogen auf das zu schützende Material.

Die erfindungsgemäßen neuen Wirkstoffe können auch in Mischung mit anderen bekannten Wirkstoffen vorliegen. Beispielsweise seien die folgenden Wirkstoffe genannt : Benzylalkoholmono(poly)hemiformal, Benzimidazolylmethylcarbamate, Tetramethylthiuramdisulfid, Zinksalze von Dialkylthiocarbamaten, 2,4,5,6-Tetrachlorisophthalonitril, Thiazolylbenzimidazol, Mercaptobenzthiazol, Trialkylzinnverbindungen, Methylenbisthiocyanate und Phenolderivate, wie 2-Phenylphenol, (2,2'-Dihydroxy-5,5'-dichlor)-diphenylmethan, 3-Methyl-4-chlor-phenol und quaternäre Ammoniumsalze.

## Herstellungsbeispiele

### Beispiel 1

70 g Bis-cyanethyl-sulfid wurden in 160 ml Dichlormethan gelöst. Dazu tropfte man zunächst bei 5 bis 10 °C, später bei 30 °C 135 g Sulfurylchlorid. Danach wurde eingeengt, mit Wasser gewaschen und an Kieselgel chromatographiert. Nach Einengen und Umkristallisieren aus Ethanol erhielt man 16 g (1-Chlor-2-cyanovinyl)-(2-cyano-ethyl)-sulfid, Schmp. 53 bis 57 °C.

$^1$H-NMR : δ = 2,6-2,9 (2H), 3,1-3,4 (2H), 5,8 (1H).

### Beispiel 2

Auf dem gleichen Wege erhielt man mit 203 g Sulfurylchlorid 23 g (1,2-Dichlor-2-cyano-vinyl)-(2-cyano-ethyl)-sulfid als Öl, $^1$H-NMR : δ = 2,7-2,9 (2H), 3,2-3,5 (2H).

### Beispiel 3

Auf dem gleichen Wege erhielt man mit 270 g Sulfurylchlorid 11,8 g (1-Chlor-2-cyano-vinyl)-(2,2-dichlor-2-cyano-ethyl)-sulfid als Öl,
$^1$H-NMR : δ-3,9-4,1 (2H), 5,8-6,0 (1H).

Beispiel 4

258 g Bis-cyanethyl-sulfid wurden in 0,9 l Dichlormethan gelöst und 938 g Kaliumcarbonat zugesetzt. Bei 5 °C wurden 905 g Chlor eingeleitet. Danach wurde gefiltert, eingeengt und chromatographiert. Man erhielt 110 g (1,2-Dichlor-2-cyano-vinyl)-(2,2-dichlor-2-cyano-ethyl)-sulfid als Öl,
$^1$H-NMR : δ = 4,0-4,2.

Beispiel 5

133 g (1,2-Dichlor-2-cyano-vinyl)-(2,2-dichlor-2-cyanoethyl)-sulfid wurden in 350 ml Tetrahydrofuran gelöst und bei 5 bis 10 °C 53,6 g Triethylamin zugetropft. Man saugte das ausgefallene Triethylaminhydrochlorid ab, engte das Filtrat ein und destillierte (Kp. 120 bis 145 °C/2,5 mbar). Man erhielt 66,3 g (1,2-Dichlor-2-cyano-vinyl)-(2-chlor-2-cyano-vinyl)-sulfid als Öl, $^1$H-NMR : δ = 7,1-7,4. Daraus kristallisierte ein reines Stereoisomer aus, Schmp. 58 bis 60 °C, $^1$H-NMR : δ = 7,1.

Beispiel 6

Lösungen von 350 g 2,3-Dichloracrylnitril in 0,75 l Dichlormethan und 241 g Natriumhydrogencarbonat in 1,6 l Wasser wurden vereinigt. Unter heftigem Rühren wurde Schwefelwasserstoff bis zur Sättigung eingeleitet, 5 h bei Raumtemperatur gerührt, nochmals mit Schwefelwasserstoff gesättigt und weitere 12 h gerührt. Die organische Phase wurde abgetrennt und eingeengt. Es hinterblieben 184 g Bis-(2-chlor-2-cyano-vinyl)-sulfid als kristalliner Stoff, Schmp. 70 bis 80 °C, $^1$H-NMR : δ = 7,2-7,6.

Beispiel 7

180 g Bis-(2-chlor-2-cyano-vinyl)-sulfid wurden in 2,6 l Trichlormethan gelöst, 180 g Kaliumcarbonat und 50 g Trioctylmethylammoniumchlorid (technisch) zugesetzt, und während 2 d wurde ein langsamer Chlorstrom durchgeleitet. Die Lösung wurde mit Wasser gewaschen und eingeengt. Der ölige Rückstand enthielt den Phasentransferkatalysator und 282 g Bis-(1,2,2-trichlor-2-cyano-ethyl)-sulfid, MS : m/e = 344/346/348/350 ($M^+$).

Beispiel 8

Der Rückstand aus Beispiel 7 wurde in 0,5 l Dichlormethan gelöst und 150 g Pyridin bei 0 bis 5 °C zugetropft. Man rührte noch 4 h, wusch mit Wasser, engte ein, nahm in Toluol auf und filtrierte über Kieselgel. Das Filtrat wurde eingeengt und destilliert (Kp. 165-172 °C/18 mbar). Man erhielt 81 g Bis-(1,2-dichlor-2-cyano-vinyl)-sulfid als Öl.
IR : $\nu_{C \equiv N}$ = 2 220, 2 205 $cm^{-1}$ · MS : m/e = 272/274/276/278 ($M^+$).
Beim Versetzen des Öles mit Leichtbenzin (Siedebereich 40-80 °C) kristallisierte ein reines Stereoisomeres aus (Schmp. 87 °C).

Beispiel 8a

290 g Bis-(2-chlor-2-cyano-vinyl)-sulfid wurden in 1 l Dichlormethan gelöst und 11,2 g Pyridin zugesetzt. Bei Raumtemperatur wurde während 48 Stunden ein langsamer Chlorstrom durchgeleitet. Die Lösung wurde mit verdünnter Salzsäure und Wasser gewaschen und eingeengt. Aus dem Rückstand wurden bei 0,2 mbar und 100 °C Sumpftemperatur Verunreinigungen herausdestilliert. Es hinterblieben 344,6 g Bis-(1,2-dichlor-2-cyano-vinyl)-sulfid als Öl. Beim Versetzen mit Leichtbenzin (Siedebereich 40 bis 80 °C) trat Kristallisation ein. Die Kristalle waren ein Gemisch der drei möglichen Stereoisomeren und schmolzen bei 69 bis 71 °C.

Beispiel 9

3,3 g (1,2-Dichlor-2-cyano-vinyl)-(2-chlor-2-cyano-vinyl)-sulfid wurden in 20 ml Dichlorethan gelöst, 2 g Kaliumcarbonat zugesetzt und bei Raumtemperatur 2,15 g Sulfurylchlorid in 10 ml Dichlorethan zugetropft. Es wurde 12 h bei 70 °C gerührt, filtriert, mit Wasser gewaschen, eingeengt und das resultierende Öl chromatographiert. Man erhielt 1,7 g Bis-(1,2-dichlor-2-cyano-vinyl)-sulfid als Öl, identisch mit dem Produkt aus Beispiel 8.

Beispiel 10

35 g Bis-cyanethyl-sulfid und 20 g Pyridin wurden in 0,5 l Dichlormethan gelöst und 138 g Kaliumcarbonat zugesetzt. Danach wurden unter Eiskühlung 110 g Chlor eingeleitet. Man ließ auf Raumtemperatur kommen und rührte 3 h, wobei ein schwacher Chlorstrom durch den Ansatz geleitet wurde. Schließlich wurde auf 40 °C erwärmt und weiter Chlor eingeleitet, bis insgesamt 250 g Chlor zugesetzt worden waren. Der Ansatz wurde gefiltert, mit Wasser gewaschen, eingeengt und destilliert (Kp. 110 bis 140 °C/0,3 mbar). Man erhielt 10,6 g eines Gemisches von (1,2-Dichlor-2-cyano-vinyl)-(2-chlor-2-cyano-vinyl)-sulfid und Bis-(1,2-dichlor-2-cyano-vinyl)-sulfid.

## Beispiel 11

28 g Bis-cyanoethyl-sulfid wurden in 250 ml Dichlorethan gelöst und 110 g Kaliumcarbonat zugesetzt. Dann wurden 64 g Brom bei 20 bis 30 °C zugetropft. Man rührte 16 h bei 40 °C, setzte weitere 40 g Kaliumcarbonat zu und rührte 16 h bei 60 °C. Der Ansatz wurde gefiltert, mit Wasser gewaschen, eingeengt und chromatographiert. Man erhielt 5,5 g (2-Brom-2-cyano-vinyl)-(2-cyano-ethyl)-sulfid als Öl,
$^1$H-NMR : δ = 2,6-3,0 (2H), 3,0-3,4 (2H), 7,5-7,9 (1H).

## Beispiel 12

17,3 g (1-Chlor-2-cyano-vinyl)-(2-cyano-ethyl)-sulfid wurden in 100 ml Dichlorethan gelöst und 9,8 g Natriumacetat zugesetzt. Dann wurden 16 g Brom bei 20 °C zugetropft. Die organische Phase wurde mit Wasser gewaschen, eingeengt und chromatographiert. Man erhielt 10,2 g (2-Brom-1-chlor-2-cyano-vinyl)-(2-cyano-ethyl)-sulfid als Öl,
$^1$H-NMR : δ = 2,6-2,9 (2H), 3,1-3,5 (2H).

## Beispiel 13

26 g (1,2-Dichlor-2-cyano-vinyl)-(2,2-dichlor-2-cyano-ethyl)-sulfid wurden mit 80 ml konzentrierter Salzsäure und 80 ml Essigsäure 30 Minuten auf 50 °C erhitzt. Man engte ein, nahm in Dichlormethan auf, wusch mit Natriumhydrogencarbonat-Lösung, trocknete die Dichlormethan-Phase und engte ein. Man erhielt 18 g 2,2-Dichlor-3-(1,2-dichlor-2-cyano-vinyl)-thio-propionsäureamid als harzartiges Öl,
$^1$H-NMR : δ = 3,8-4,2 (2H), 6,5-7,3 (2H).

## Beispiel 14

38,6 g (1,2-Dichlor-2-cyano-vinyl)-(2,2-dichlor-2-cyanoethyl)-sulfid wurden mit 116 ml konzentrierter Salzsäure und 116 ml Essigsäure 2,5 h auf 70 °C erhitzt. Man engte ein, nahm mit Dichlormethan auf und wusch mit Natriumhydrogencarbonat-Lösung. Die wäßrige Lösung wurde mit Salzsäure angesäuert und mit Dichlormethan extrahiert. Nach Einengen der Dichlormethanphase erhielt man 26,6 g 2,2-Dichlor-3-(1,2-dichlor-2-cyano-vinyl)-thio-propionsäure als zähes Öl,
$^1$H-NMR : δ = 3,8-4,2 (2H), 9,7 (1H).

## Beispiel 15

26,6 g 2,2-Dichlor-3-(1,2-dichlor-2-cyano-vinyl)-thio-propionsäure wurden in 200 ml Wasser suspendiert und 7,6 g Natriumhydrogencarbonat zugegeben. Die wäßrige Lösung wurde mit Dichlormethan gewaschen und eingeengt. Man erhielt 28 g Natriumsalz,
$^1$H-NMR ($d_6$-DMSO) : δ = 3,9-4,2.

## Beispiele 16 bis 22

Entsprechend Beispiel 13 bis 15 wurden die folgenden Verbindungen hergestellt und durch Kernresonanzdaten charakterisiert :

| Bsp. Nr. | Formel | $^1$H-NMR: δ = |
|---|---|---|
| 16 | $H_2N-CO-CCl_2-CH_2-S-CCl=CH-CN$ | 3,8-4,2 (2H), 5,7-5,9 (1H), 6,5-7,2 (2H) |
| 17 | $H_2N-CO-CH_2-CH_2-S-CCl=CH-CN$ | 2,4-2,9 (2H), 3,0-3,5 (2H), 5,7 (1H), 6,2-6,7 (2H) |

Tabelle (Fortsetzung)

| Bsp. Nr. | Formel | $^1$H-NMR: $\delta$ = |
|---|---|---|
| 18 | $H_2N-CO-CH_2-CH_2-S-CCl=CCl-CN$ | 2,4-2,8 (2H), 3,1-3,6 (2H), 5,5-6,5 (2H) |
| 19 | $HOOC-CH_2-CH_2-S-CCl=CH-CN$ | 2,6-2,9 (2H), 3,0-3,5 (2H), 5,6-5,7 (1H), 10,7 (1H) |
| 20 | $HOOC-CH_2-CH_2-S-CCl=CCl-CN$ | 2,6-2,9 (2H), 3,1-3,5 (2H), 11,3 (1H) |
| 21 | $HOOC-CCl_2-CH_2-S-CCl=CH-CN$ | 3,9-4,1 (2H), 5,7-5,9 (1H), 9,7 (1H) |
| 22 | $Na^{\oplus\ominus}O-CO-CCl_2-CH_2-S-CCl=CH-CN$ | 4,0-4,2 (2H), 5,9-6,1 (1H), ($CD_3OD$) |

## Anwendungsbeispiele

### Beispiel 23

Zum Nachweis der Wirksamkeit gegen Pilze werden die minimalen Hemm-Konzentrationen (MHK) von erfindungsgemäßen Wirkstoffen bestimmt :

Ein Agar, der aus Bierwürze und Pepton hergestellt wird, wird mit erfindungsgemäßen Wirkstoffen in Konzentrationen von 0,1 mg/l bis 5 000 mg/l versetzt. Nach Erstarren des Agars erfolgt Kontamination mit Reinkulturen der in der Tabelle aufgeführten Testorganismen. Nach 2-wöchiger Lagerung bei 28 °C und 60 bis 70 % relativer Luftfeuchtigkeit wird die MHK bestimmt. MHK ist die niedrigste Konzentration an Wirkstoff, bei der keinerlei Bewuchs durch die verwendete Mikrobenart erfolgt ; sie ist in der nachstehenden Tabelle angegeben.

Als Vergleichssubstanz dient α-Chlor-β-phenylsulfonylacrylnitril, das als Mikrobizid zum Schutze technischer Materialien verwendet wird (DOS 25 00 265).

(Siehe Tabelle I Seite 11 f.)

Tabelle I

Angabe der MHK-Werte in mg/l bei der Einwirkung erfindungsgemäßer Substanzen auf Pilze

| Testorganismen | Wirkstoff gemäß Beispiel 3 | 5 | 4 | 8 | 2 | 9 | α-Chlor-ß-phenyl-sulfonyl-acryl-nitril als Vergleichssubstanz |
|---|---|---|---|---|---|---|---|
| Alternaria tenuis | 10 | 20 | 5 | 2 | | 0,75 | |
| Aspergillus niger | 20 | 2 | 10 | 2 | <20 | 2,5 | 50 |
| Aureobasidium pullulans | 10 | 5 | 5 | 1 | | 1 | |
| Chaetomium globosum | 15 | 2 | 10 | 2 | <20 | 2,5 | 50 |
| Coniophora puteana | 1 | <0,1 | 0,5 | <0,1 | | 0,5 | |
| Lentinus tigrinus | 5 | 0,5 | 2 | 0,5 | | 1 | |
| Penicillium glaucum | 20 | 1 | 10 | 1 | | 2,5 | 35 |
| Polyporus versicolor | 10 | 1 | 5 | 1 | | 1 | |
| Sclerophoma pityophila | 5 | 0,5 | 2 | 0,5 | 20 | 0,75 | |
| Trichoderma viride | 100 | 10 | 50 | 10 | | 10 | |

Beispiel 24 (Wirkung gegen Schleimorganismen)

Erfindungsgemäße Substanzen werden in Konzentrationen von jeweils 0,1 bis 100 mg/l in Allens Nährlösung (Arch. Mikrobiol. *17*, 34 bis 53 (1952)), die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1 % Caprolactam enthält, in wenig Aceton gelöst, zur Anwendung gebracht. Kurz vorher wird die Nährlösung mit Schleimorganismen (ca. $10^6$ Keime/ml), die aus bei der Polyamid-Herstellung verwendeten Spinnwasser-Kreisläufen isoliert wurden, infiziert. Nährlösungen, die die minimale Hemmkonzentration (MHK) oder größere Wirkstoffkonzentrationen aufweisen, sind nach 3-wöchiger Kultur bei Raumtemperatur noch völlig klar, d. h. die in wirkstofffreien Nährlösungen nach 3 bis 4 Tagen bemerkbare starke Vermehrung der Mikroben und Schleimbildung unterbleibt.

Tabelle II

MHK-Werte in mg/l bei der Einwirkung der unten angegebenen Substanzen auf Schleimorganismen

| Wirkstoff gemäß Beispiel | MHK in mg/l |
|---|---|
| 3 | 0,35 |
| 5 | 0,35 |
| 4 | 0,35 |
| 2 | 2 |
| 1 | 0,15 |

Beispiel 25

Wirkung gegen Bakterien

Ein Agar, der als Nährmedien Bouillon enthält, wird mit erfindungsgemäßen Wirkstoffen in

Konzentrationen von 1 bis 5 000 mg/l versetzt. Darauf infiziert man das Nährmedium jeweils mit den in Tabelle III aufgeführten Testorganismen und hält das infizierte Medium 2 Wochen bei 28 °C und 60 bis 70 % rel. Luftfeuchtigkeit. Die MHK ist die niedrigste Konzentration an Wirkstoff, bei der keinerlei Bewuchs durch die verwendete Mikrobenart erfolgt. Die MHK-Werte sind in Tabelle III wiedergegeben.

Tabelle III

Angabe der MHK-Werte in mg/l bei der Einwirkung der unten angegebenen Wirkstoffe auf Bakterien.

| Testorganismen | Wirkstoff gemäß Beispiel | | | | | | α-Chlor-ß-phenyl-sulfonyl-acrylnitril als Vergleichssubstanz |
|---|---|---|---|---|---|---|---|
| | 3 | 5 | 4 | 8 | 2 | 9 | |
| Escherichia coli | 200 | 20 | 50 | 50 | 200 | 25 | 200 |
| Staphylococcus aureus | 50 | 10 | 50 | < 20 | 35 | 10 | 500 |

Beispiel 26

Eine Mischkultur von Grün-, Blau-, Braun- und Kieselalgen (Stichococcus bacillaris Naegeli, Euglena gracilis Klebs, Chlorella pyrenoidosa Chick, Phormidium foveolarum Gomont, Oscillatoria geminata Meneghini und Phaedodactylum tricornutum Bohlin) wird unter Durchperlen von Luft in Allens Nährlösung (Arch. Mikrobiol. *17*, 34 bis 53 (1952)), die auf 4 l steriles Wasser 0,2 g Ammoniumchlorid, 4,0 g Natriumnitrat, 1,0 g Dikaliumhydrogenphosphat, 0,2 g Calciumchlorid, 2,05 g Magnesiumsulfat und 0,02 g Eisenchlorid enthält, gegeben. Nach 2 Wochen ist die Nährlösung durch intensives Algenwachstum tief grünblau gefärbt. Das Absterben der Algen nach Zugabe erfindungsgemäßer Wirkstoffe erkennt man an dem Entfärben der Nährlösung.

Tabelle IV

Algen-abtötende Konzentrationen (mg/l) der unten angegebenen Substanzen

| Wirkstoff gemäß Beispiel | abtötende Konzentration in mg/l |
|---|---|
| 3 | 25 |
| 5 | 25 |
| 4 | 25 |
| 2 | 50 |
| 1 | 100 |

Beispiel 27

Prüfung von (1,2-Dichlor-2-cyano-vinyl)-(2-chlor-2-cyano-vinyl)-sulfid gemäß Beispiel 5 als Konservierungsstoff für Kühlschmiermittel.

Ein Kühlschmiermittel auf Mineralölbasis wird mit 1 % des Wirkstoffs als Konservierungsstoff versetzt.

5 %ige Gebrauchsverdünnungen des Kühlschmiermittels werden während 3 Monaten täglich massiv mit Mikroben (Bakterien, Hefen, Schimmelpilzen), isoliert aus mikrobiell verdorbenen Kühlschmiermittelgebrauchsverdünnungen, kontaminiert.

Die Gebrauchsverdünnungen sind am Ende der Prüfzeit noch keimfrei ; sie sind also zuverlässig konserviert.

**Patentansprüche**

1. Halogenierte Sulfide der Formel

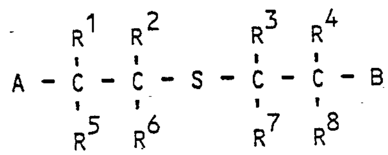

in der

$R^1$ bis $R^8$ gleich oder verschieden sind und Wasserstoff oder Halogen bedeuten, wobei mindestens einer der Reste $R^1$ bis $R^8$ für Halogen steht, und wobei gegebenenfalls $R^1$ mit $R^2$ und/oder $R^3$ mit $R^4$ eine olefinische Bindung bilden können, und

A und B gleich oder verschieden sind und Nitril, Carboxyl, Carboxylat oder Carboxamid bedeuten.

2. Halogenierte Sulfide nach Anspruch 1 der Formel

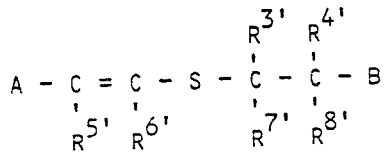

in der

$R^{3'}$ bis $R^{8'}$ gleich oder verschieden sind und Wasserstoff, Chlor oder Brom bedeuten, wobei mindestens einer der Reste $R^{3'}$ bis $R^{8'}$ für Chlor oder Brom steht und wobei $R^{3'}$ mit $R^{4'}$ gegebenenfalls eine olefinische Bindung bilden kann, und

A und B gleich oder verschieden sind und Nitril, Carboxyl, Carboxylat oder Carboxamid bedeuten.

3. Verfahren zur Herstellung von halogenierten Sulfiden der Formel

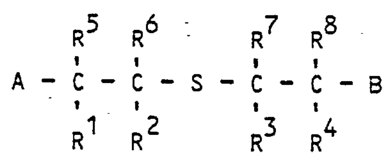

in der

$R^1$ bis $R^8$ gleich oder verschieden und Wasserstoff oder Halogen bedeuten, wobei mindestens einer der Reste $R^1$ bis $R^8$ für Halogen steht, und wobei gegebenenfalls $R^1$ mit $R^2$ und/oder $R^3$ mit $R^4$ eine olefinische Bindung bildet, und

A und B gleich oder verschieden sind und Nitril, Carboxyl, Carboxylat oder Carboxamid bedeuten, dadurch gekennzeichnet, daß man Bis-(cyanethyl)-sulfid mit einem Halogenierungsmittel, gegebenenfalls in Gegenwart eines säurebindenden Mittels, umsetzt und dann in an sich bekannter Weise aufarbeitet.

4. Verfahren nach Anspruch 3, dadurch gekennzeichnet, daß man in einer ersten Stufe das Bis-(cyanethyl)-sulfid mit dem Halogenierungsmittel umsetzt und in einer zweiten Stufe das säurebindende Mittel zufügt.

5. Verfahren zur Herstellung von halogenierten Sulfiden der Formel

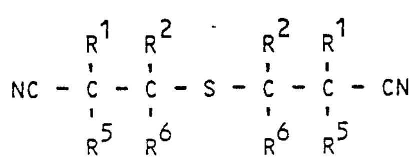

in der $R^1$, $R^2$, $R^5$ und $R^6$ gleich oder verschieden sind und Wasserstoff oder Halogen bedeuten, wobei mindestens einer der Reste $R^1$, $R^2$, $R^5$ oder $R^6$ für Halogen steht, und wobei gegebenenfalls $R^1$ mit $R^2$ eine olefinische Bindung bilden kann,
dadurch gekennzeichnet, daß man in einem ersten Reaktionsschritt ein Sulfid der Formel

Me 2/n S

in der

Me Wasserstoff, Alkali, Erdalkali oder Ammonium bedeutet und

n für die Wertigkeit des Restes Me steht,

mit 3-Halogen-acrylnitrilen der Formel

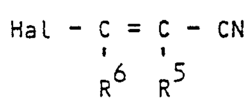

Hal für Chlor oder Brom steht,

$R^5$ und $R^6$ die obengenannte Bedeutung haben,

gegebenenfalls in Gegenwart einer Base umsetzt, dann gegebenenfalls in einem zweiten Reaktionsschritt das Reaktionsprodukt halogeniert, dann gegebenenfalls in einem dritten Reaktionsschritt mit einem säurebindenden Mittel umsetzt und dann gegebenenfalls in einem vierten Reaktionsschritt wieder halogeniert und die einzelnen Reaktionsschritte gegebenenfalls jeweils in an sich bekannter Weise aufarbeitet.

6. Verfahren nach Anspruch 5, dadurch gekennzeichnet, daß man zwei oder mehrere der Reaktionsschritte zusammenfaßt.

7. Mikrobizides Mittel, enthaltend halogenierte Sulfide der Formel

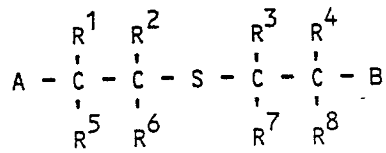

in der

$R^1$ bis $R^8$ gleich oder verschieden sind und Wasserstoff oder Halogen bedeuten, wobei mindestens einer der Reste $R^1$ bis $R^8$ für Halogen steht, und wobei gegebenenfalls $R^1$ mit $R^2$ und/oder $R^3$ mit $R^4$ eine olefinische Bindung bilden können, und

A und B gleich oder verschieden sind und Nitril, Carboxyl, Carboxylat oder Carboxamid bedeuten.

8. Verwendung von mikrobiziden Mitteln nach Anspruch 7 zum Schutz technischer Materialien vor mikrobieller Zerstörung.

9. Verwendung nach Anspruch 8, dadurch gekennzeichnet, daß man 0,001 bis 5 Gew.-% des Wirkstoffes bezogen auf das zu schützende Material einsetzt.

**Claims**

1. Halogenated sulphides of the formula

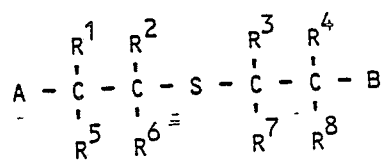

in which

$R^1$ to $R^8$ are identical or different and denote hydrogen or halogen, at least one of the radicals $R^1$ to $R^8$ representing halogen, and it optionally being possible for $R^1$ together with $R^2$ and/or $R^3$ together with $R^4$ to form an olefinic bond, and

A and B are identical or different and denote nitrile, carboxyl, carboxylate or carboxamide.

2. Halogenated sulphides according to Claim 1 of the formula

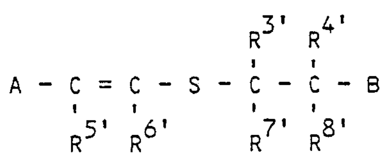

in which

$R^{3'}$ to $R^{8'}$ are identical or different and denote hydrogen, chlorine or bromine, at least one of the radicals $R^{3'}$ to $R^{8'}$ representing chlorine or bromine and it optionally being possible for $R^{3'}$ together with $R^{4'}$ to form an olefinic bond, and

A and B are identical or different and denote nitrile, carboxyl, carboxylate or carboxamide.

3. Process for the preparation of halogenated sulphides of the formula

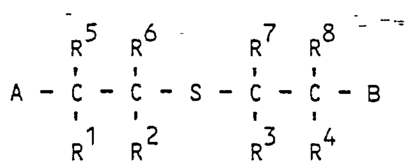

in which

$R^1$ to $R^8$ are identical or different and denote hydrogen or halogen, at least one of the radicals $R^1$ to $R^8$ representing halogen, and $R^1$ together with $R^2$ and/or $R^3$ together with $R^4$ optionally forming an olefinic bond, and

A and B are identical or different and denote nitrile, carboxyl, carboxylate or carboxamide, characterised in that bis-(cyanoethyl) sulphide is reacted with a halogenating agent, if appropriate in the presence of an acid-binding agent, and the mixture is then worked up in a manner known *per se.*

4. Process according to Claim 3, characterised in that the bis-(cyanoethyl) sulphide is reacted with the halogenating agent in a first stage and the acid-binding agent is added in a second stage.

5. Process for the preparation of halogenated sulphides of the formula

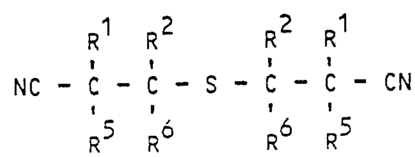

in which $R^1$, $R^2$, $R^5$ and $R^6$ are identical or different and denote hydrogen or halogen, at least one of the radicals $R^1$, $R^2$, $R^5$ or $R^6$ representing halogen, and it optionally being possible for $R^1$ together with $R^2$ to form an olefinic bond,

characterised in that, in a first reaction step, a sulphide of the formula

$$Me_{2/n}S$$

in which

Me denotes hydrogen, an alkali metal, an alkaline earth metal or ammonium and

n represents the valency of the radical Me,

is reacted with 3-halogeno-acrylonitriles of the formula

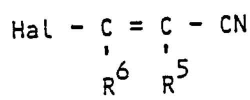

Hal represents chlorine or bromine,

$R^5$ and $R^6$ have the abovementioned meaning,

if appropriate in the presence of a base, the reaction product is then halogenated, if appropriate, in a second reaction step, the product is then reacted with an acid-binding agent, if appropriate, in a third reaction step and the product is then halogenated again, if appropriate, in a fourth reaction step, and the individual reaction steps are worked up, if appropriate, in each case in a manner which is known *per se.*

6. Process according to Claim 5, characterised in that two or more of the reaction steps are combined.

7. Microbicidal agent, containing halogenated sulphides of the formula

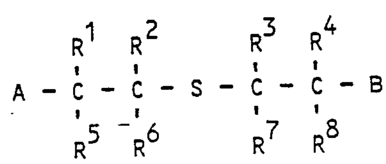

in which

$R^1$ to $R^8$ are identical or different and denote hydrogen or halogen, at least one of the radicals $R^1$ to $R^8$ representing halogen, and it optionally being possible for $R^1$ together with $R^2$ and/or $R^3$ together with $R^4$ to form an olefinic bond, and

A and B are identical or different and denote nitrile, carboxyl, carboxylate or carboxamide.

8. Use of microbicidal agents according to Claim 7 for protecting industrial materials against microbial destruction.

9. Use according to Claim 8, characterised in that 0.001 to 5 % by weight of the active compound is employed, based on the material to be protected.

**Revendications**

1. Sulfures halogénés, de formule :

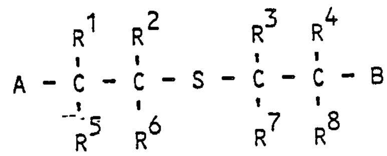

dans laquelle

$R^1$ à $R^8$ sont identiques ou différents et représentent chacun un atome d'hydrogène ou d'halogène, au moins l'un des restes $R^1$ à $R^8$ représentant un atome d'halogène, et éventuellement $R^1$ peut former avec $R^2$ et/ou $R^3$ peut former avec $R^4$ une liaison oléfinique, et

A et B sont identiques ou différents et représentent chacun un groupe nitrile, carboxyle, carboxylate ou carboxamide.

2. Sulfures halogénés selon la revendication 1, de formule :

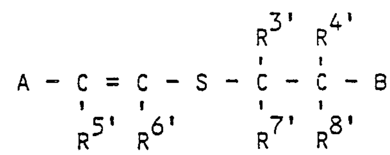

dans laquelle

$R^{3'}$ à $R^{8'}$ sont identiques ou différents et représentent chacun un atome d'hydrogène, de chlore ou de brome, et au moins l'un des restes $R^{3'}$ à $R^{8'}$ représente un atome de chlore ou de brome et $R^{3'}$ peut former avec $R^{4'}$ éventuellement une liaison oléfinique, et

A et B sont identiques ou différents et représentent chacun un groupe nitrile, carboxyle, carboxylate ou carboxamide.

3. Procédé pour préparer des sulfures halogénés de formule :

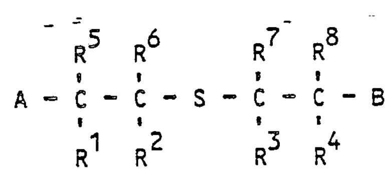

dans laquelle

$R^1$ à $R^8$ sont identiques ou différents et représentent chacun un atome d'hydrogène ou d'halogène, au moins l'un des restes $R^1$ à $R^8$ représentant un atome d'halogène, et éventuellement $R^1$ peut former avec $R^2$ et/ou $R^3$ peut former avec $R^4$ une liaison oléfinique, et

A et B sont identiques ou différents et représentent chacun un groupe nitrile, carboxyle, carboxylate ou carboxamide,

procédé caractérisé en ce qu'on fait réagir du sulfure de bis-(cyanéthyle) avec un agent d'halogénation, éventuellement en présence d'un agent de fixation des acides, puis l'on effectue de façon connue un traitement final de préparation.

4. Procédé selon la revendication 3, caractérisé en ce qu'on fait réagir dans la première étape le sulfure de bis-(cyanéthyle) avec l'agent d'halogénation et l'on introduit dans une seconde étape l'agent de fixation des acides.

5. Procédé pour préparer des sulfures halogénés de formule :

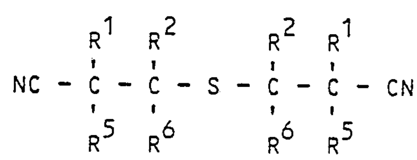

dans laquelle

$R^1$, $R^2$, $R^5$ et $R^6$ sont identiques ou différents et représentent un atome d'hydrogène ou d'halogène, au moins l'un des restes $R^1$, $R^2$, $R^5$ ou $R^6$ étant un atome d'halogène, et éventuellement $R^1$ peut former avec $R^2$ une liaison oléfinique,

procédé caractérisé en ce que, dans une première étape de réaction, on fait réagir, éventuellement en présence d'une base, un sulfure de formule :

Me 2/n S

dans laquelle

Me représente un atome d'hydrogène, de métal alcalin, du métal alcalino-terreux ou un groupe ammonium,

n a la valence du reste Me,

avec des 3-halogéno-acrylonitriles de formule :

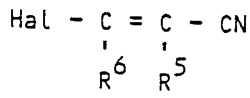

dans laquelle

Hal représente le chlore ou le brome,

$R^5$ et $R^6$ ont le sens indiqué ci-dessus,

puis, éventuellement dans une seconde étape de réaction, on halogène le produit de la réaction puis, éventuellement dans une troisième étape de réaction, on fait réagir avec un agent de fixation des acides, puis, éventuellement dans une quatrième étape de réaction, on halogène à nouveau et, dans les diverses étapes de réaction, on effectue éventuellement à chaque fois, de façon connue en soi, le traitement final de préparation.

6. Procédé selon la revendication 5, caractérisé en ce qu'on regroupe deux ou plus de deux des étapes de réaction.

7. Produit microbicide, contenant des sulfures halogénés de formule :

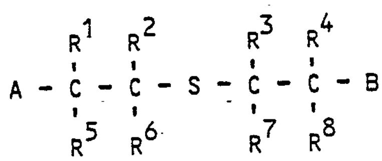

dans laquelle

$R^1$ à $R^8$ sont identiques ou différents et représentent un atome d'hydrogène ou d'halogène, au moins l'un des restes $R^1$ à $R^8$ représentant un atome d'halogène, et éventuellement $R^1$ peut former avec $R^2$ et/ou $R^3$ peut former avec $R^4$ une liaison oléfinique, et

A et B sont identiques ou différents et représentent chacun un groupe nitrile, carboxyle, carboxylate ou carboxamide.

8. Utilisation de produits microbicides selon la revendication 7 pour protéger des matières techniques contre une destruction par des microbes.

9. Utilisation selon la revendication 8, caractérisée en ce qu'on utilise 0,001 à 5 % en poids de la substance active par rapport à la matière à protéger.